Europäisches Patentamt

European Patent Office

Office européen des brevets

(11) Publication number: **0 218 330**
A1

## EUROPEAN PATENT APPLICATION

(21) Application number: 86306138.8

(22) Date of filing: 08.08.86

(51) Int. Cl.⁴: **A 61 F 7/10**, A 61 D 9/00

(30) Priority: 12.08.85 GB 8520163

(43) Date of publication of application: 15.04.87
Bulletin 87/16

(84) Designated Contracting States: AT BE CH DE FR IT LI LU NL SE

(71) Applicant: Williams, Davina Mary, Bishops Croft Farm Shurton Stogursey, Bridgwater Somerset TA5 IQE (GB)

(72) Inventor: Williams, Davina Mary, Bishops Croft Farm Shurton Stogursey, Bridgewater Somerset, TA5 1QE (GB)
Inventor: Williams, John Richard, Bishops Croft Farm Shurton Stogursey, Bridgewater Somerset, TA5 1QE (GB)

(74) Representative: Jones, Michael Raymond et al, HASELTINE LAKE & CO. Hazlitt House 28 Southampton Buildings Chancery Lane, London WC2A 1AT (GB)

(54) Hosing pad.

(57) There is disclosed a hosing pad (1), preferably for the treatment of swellings on an animal's leg. The hosing pad (1) comprises a hollow member (6) adapted to be secured to a region of the body to be treated, the hosing pad (1) including an inlet (5) for attachment to a water supply and a plurality of outlets (7) from which water is discharged from the hollow member (6) onto the region to be treated. The hosing pad (1) may further include a layer of an absorbent material (8) as an intermediate layer between the hollow member (6) and the region to be treated.

## HOSING PAD

This relates to a hosing pad by which water may be dispensed over a region of a human or animal body to be treated.

A swelling on an animal's leg, particularly a horse's leg, is normally treated by hosing down the swelling with cold water. This is a time consuming, manual operation.

According to the present invention there is provided a hosing pad comprising a hollow member for attachment to a region of a human or animal body to be treated, the hollow member being provided with an inlet for attachment to a water source and a plurality of outlets by which, in use, water is discharged from the hollow body onto said region to be treated.

Preferably, the hosing pad further comprises a layer of absorbent material, for example foam material, provided over the plurality of outlets so as to act as an intermediate layer between the hollow member and the region of the human or animal body to be treated. This layer of absorbent material assists in keeping the water discharged from the outlets in contact with the region of the body to be treated.

The hosing pad may also include securing means by which the hollow member is securable to the region to be treated. This securing means may comprise one or more buckles and corresponding straps or complimentary male and female VELCRO pieces arranged such that the hosing pad can be wrapped around a limb of a human or animal body and secured in place.

The hollow member may be supported, for example by stitching, on a backing membrane, which is preferably water proof. The layer of absorbent material may lay across the whole area of the backing membrane and the securing means may be fixed to the backing membrane.

Preferably, the device of the present invention is adapted to be applied to a limb of an animal, such as a horse.

In use, the hosing pad supplies a regular supply of water to the affected region. The hosing pad is strapped to the area to be treated and the inlet is connected to a water supply by, for example, a hose.

The hollow member may be made of a waterproof plastic material (e.g. PVC). The backing membrane may be made of the same material.

For a better understanding of the present invention and to show how the same may be carried into effect, reference will now be made, by way of example to the accompanying drawings in which;

Figure 1 illustrates one side of a hosing pad in accordance with the present invention;

Figure 2 shows the reverse side of the hosing pad shown in Figure 1; and

Figure 3 shows a view of the other side of the hosing pad shown in Figure 1 further including an absorbent layer.

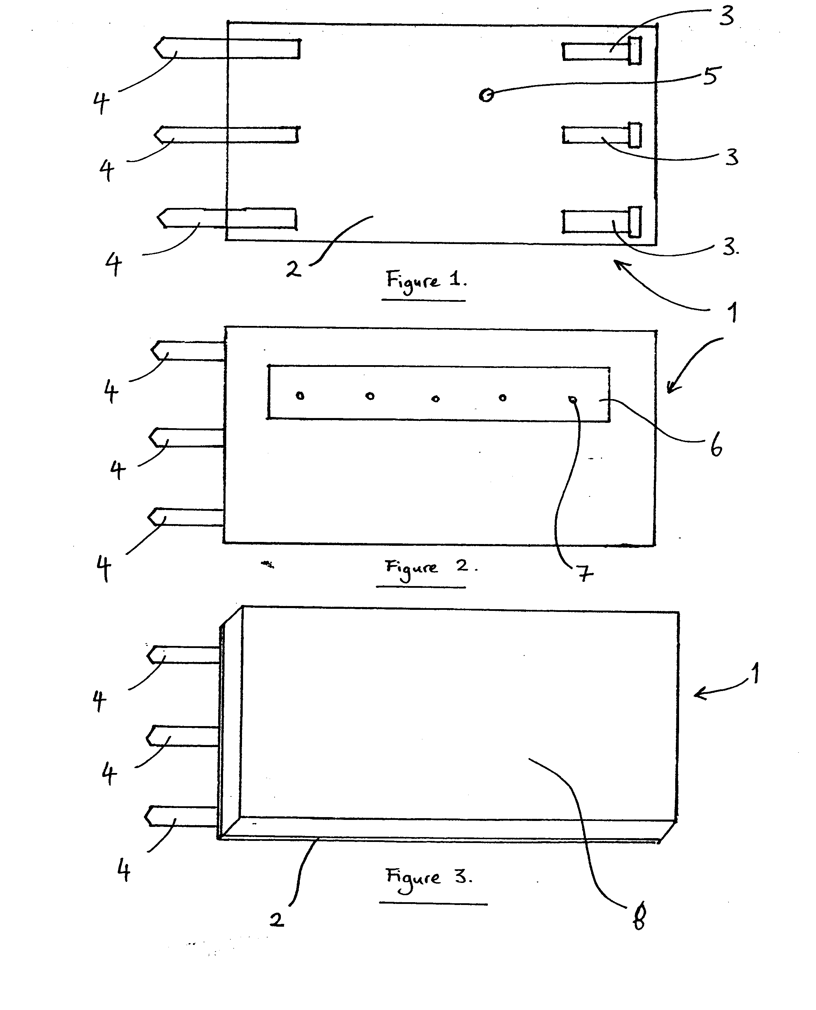

Referring to Figure 1, there is shown a hosing pad 1 comprising a backing membrane 2 to which there are fixed three buckles 3 and three corresponding straps 4. The buckles 3 and corresponding straps 4 enable the hosing pad to be secured around, for example, the limb of a horse which is to be hosed with a view to reducing swelling in the limb. Attached to the backing membrane 2 is an inlet 5, the inlet passing through the backing membrane 2 and into a hollow member 6 which is more clearly shown in Figure 2. The hollow member 6 includes a plurality of outlets 7 by which water in the hollow member 6 is discharged onto the region to be treated. The hollow member 6 is preferably sewn to the backing membrane 2. The hollow member 6 may, alternatively, or in addition, be glued to the backing membrane.

In use, the inlet 5 is attached to a water supply. Water flows into the hollow member 6 and, from there, out through the outlets 7 in the hollow member 6.

In order to retain the water discharged from the outlets in contact with the body, a layer of an absorbent material 8, such as a foam, is applied over that side of the backing membrane 2 on which the hollow member 6 is secured.

The advantage of this device is that treatment can be carried out without constant attendance of the animal being treated.

## CLAIMS

1. A hosing pad comprising a hollow member for attachment to a region of a human or a animal body to be treated, the hollow member being provided with an inlet for attachment to a water source and a plurality of outlets by which water is discharged from the hollow member onto said regions to be treated.

2. A hosing pad according Claim 1, further comprising a layer of absorbent material secured over the plurality of outlets thereby to assist in retaining water at the region to be treated.

3. A hosing pad according to Claim 2, wherein the absorbent material is a foam padding.

4. A hosing pad according to any one of Claims 1 to 3, further comprising securing means for securing the hosing pad to the region to be treated.

5. A hosing pad according to any preceding claim, wherein the hollow member is secured to a backing membrane which has a cross-sectional area greater than the cross-sectional area of the hollow member.

6. A hosing pad according to Claim 5, wherein the hollow member is sewn to the backing member.

7. A method of hosing a region of a human or animal body to be treated, comprising securing a hosing pad according to any preceding claim to the region to be treated; attaching the inlet of the hosing pad to a water supply; and permitting water to pass through the inlet and hollow member and out of the outlets onto the region to be treated.

Figure 1.

Figure 2.

Figure 3.

# EUROPEAN SEARCH REPORT

European Patent Office

## DOCUMENTS CONSIDERED TO BE RELEVANT

| Category | Citation of document with indication. where appropriate, of relevant passages | Relevant to claim | CLASSIFICATION OF THE APPLICATION (Int. Cl.4) |
|---|---|---|---|
| X | US-A-4 180 077  (McCOWAN) <br> * claim 1; figures 2, 4 * | 1 | A 61 F    7/10 <br> A 61 D    9/00 |
| A | | 4 | |
| | --- | | |
| X | DE-B-2 423 202  (MOGCK) <br> * claim 1; figures 1, 2 * | 1 | |
| A | | 4 | |
| | --- | | |
| A | DE-U-8 506 611  (OTTENHUES) <br> * claim; figure * | 1-4 | |
| | --- | | |
| A | US-A-3 905 367  (DAPCICH) <br> *   claims 1, 4, 5; figures 1-3, 5 * | 1-6 | **TECHNICAL FIELDS SEARCHED (Int. Cl.4)** <br><br> A 61 D    9/00 <br> A 61 F    7/00 |
| | ----- | | |

The present search report has been drawn up for all claims

| Place of search | Date of completion of the search | Examiner |
|---|---|---|
| BERLIN | 07-11-1986 | KANAL P K |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO Form 1503 03.82